# EUROPEAN PATENT APPLICATION

(11) **EP 3 788 937 A1**
(43) Date of publication of application: **10.03.2021**
(21) Application number: 19195914.7
(22) Date of filing: 06.09.2019
(51) Int. Cl.: A61B 1/00, A61B 1/05, H04B 10/25

(54) **SENSOR ARRANGEMENT, IMAGING DEVICE, MEDICAL DEVICE AND METHOD OF OPERATING A SENSOR ARRANGEMENT**

(71) Applicant: ams Sensors Belgium BVBA, 2600 Antwerpen (BE)
(72) Inventor: De Ridder, Tine, 2600 Antwerpen (BE); Franco, Paulo, 2600 Antwerpen (BE); Serrano Gotarredona, Rafael, 2600 Antwerpen (BE); Troxler, Thomas, 2600 Antwerpen (BE)
(74) Representative: Epping - Hermann - Fischer

(57) **Abstract**

A sensor arrangement for an endoscope with a sensor unit (1) comprises an optical sensor (2) and a transmission unit (3). A semiconductor laser (4) is connected to an optical interface (5). The transmission unit (3) is arranged to receive a sensor signal generated by the optical sensor (2) and drive the semiconductor laser (4) to provide at the optical interface (5) an optical signal representative of the sensor signal.

## Description

This invention relates to the field of imaging, such as medical imaging using a sensor or any other kind of sensor generating a large amount of data in other application areas. A proposed concept relates to a sensor arrangement, imaging device, medical device and method of operating a sensor arrangement.

Imaging techniques are used in numerous fields including consumer, communications, industrial, medical, and automotive technology. Imaging in medicine includes various diagnostic methods that provide images from the inside of the body. Important imaging procedures in medicine are ultrasound, X-ray and nuclear diagnostics, magnetic resonance imaging, computed tomography and endoscopy.

Further development of imaging technology faces challenges which often are in conflict with each other. For example, image sensors are supposed to provide ever higher resolution or higher frame rates. At the same time, however, the same sensors should be small and reliable, especially in medical applications where the sensor is inserted into the human body. For example, in endoscopy often sensor units are used in micro-camera modules. Micro-camera modules for disposable medical applications need to be both cheap and small as they typically are used just once. This poses a number of challenges on design and manufacturing.

Currently, communication from the image sensor to a base station is performed using regular electrical wires, e.g. in endoscopy to carry the data between the sensor unit and the base station. For high quality cable the cost is extremely high, which contradicts the need for disposability of the device. Cheap cables typically degrade signal integrity, making it difficult to ensure a reliable data communication channel with sufficient bit-error-rate. The crosstalk inside the cable introduces noise on the sensor supply, for example. On the sensor unit the die size may be limited so putting on-chip decoupling capacitors to keep the supply clean comes at the cost of less area available for the sensor's circuits. Putting a decoupling capacitor in the sensor unit complicates assembly as well as increasing the cost. A second problem is that the bandwidth of cheap, thin cables limits the sensor's resolution and frame rate. This becomes an increasing problem now that stacked image sensor technologies are available, enabling a large resolution image array combined with sufficient die area (on the logic die) to implement more complex image sensor functionality.

Other types of sensors such as magnetic resonance imaging, MRI, coils, or micro cameras generating a large amount of data may also need processing which is located outside the sensor itself. This requires a high-speed data interface from the sensor to a receiving unit such as an external control unit but typically not in the other direction. Similar to the situation of endoscopes cheap electrical cables typically degrade signal integrity, making it difficult to ensure a reliable data communication channel with sufficient bit-error-rate. The crosstalk inside the cable introduces as well noise on the sensor supply, which might result in performance degradation. In such sensors, typically limited space is available which prevents to add additional large capacitors to filter and clean the supply voltage.

Today there are devices in which the communication from the sensor, such as image sensors, is performed using regular purely electrical wires for the communication with a control unit. Such devices include inductive proximity sensors which use supply modulation of the supply for configuring the device. Start of communication is detected by a rising edge of the supply level at a rate faster than certain slope. A zero is transmitted as single pulse in a bit time period and a one consists of two pulses in s bit time period. Such concept may reduce the impact of crosstalk but demand more complex and thus costly data processing.

It is an objective to provide a sensor arrangement, an imaging device, a medical device and a method of operating a sensor arrangement with improved communication of sensor data and reduced cost.

These objectives are achieved by the subject matter of the independent claims. Further developments and embodiments are described in dependent claims.

It is to be understood that any feature described in relation to any one embodiment may be used alone, or in combination with other features described herein, and may also be used in combination with one or more features of any other of the embodiments, or any combination of any other of the embodiments unless described as an alternative. Furthermore, equivalents and modifications not described below may also be employed without departing from the scope of the sensor arrangement, imaging device, medical device and method of operating a sensor arrangement which are defined in the accompanying claims.

The following relates to an improved concept in the field of medical imaging using an optical sensor. The improved concept suggests a sensor arrangement for an endoscope. A sensor unit can be used for or in an endoscope and comprises an optical sensor. For example, the sensor unit may combine an image sensor, a VCSEL and a VCSEL driver into one module. A sensor's outputs can be used to drive the VCSEL to transmit the image data over an optical waveguide, such as a polymeric optical fiber, POF. This way much larger data rate can be achieved, enabling higher frame rates and/or resolution for the image sensor. An extra advantage is that communication to the sensor to configure can be done over electrical wires, separated from the medium carrying the data communication from the sensor to the receiver of the image, e.g. the optical waveguide. This may simplify controlling the sensor.

The following also relates to an improved concept in the field of imaging using a generic sensor. These concepts may include optical sensors, such as image sensor but could also be applied to different sensors such as MRI coils, for example. The improved concept suggests optical communication using communication cable with an optical waveguide, e.g. an optical fiber, POF) as physical medium for image large sensor data transmission. This solves the issue of limited bandwidth inherent to cheap, thin copper cables. In addition, signal integrity will be much better with optical communication than with copper cable or other types of electrical wiring. It prevents electromagnetic disturbances and is largely immune against them. The optical waveguide can be thin, lightweight and flexible. These are all benefits not only for medical endoscopy applications. While the optical waveguide in itself might not be cheaper, the fact that it enables higher data rates will be beneficial for various types of sensor applications.

In at least one embodiment a sensor arrangement for an endoscope comprises a sensor unit. The sensor unit comprises an optical sensor and a transmission unit. A semiconductor laser is connected to an optical interface. For example, the sensor unit provides a framework to electrically interconnect its components. The sensor unit may be implemented as one or more integrated circuits, a microchip or circuit board with the optical sensor, transmission unit, semiconductor laser and optical interface integrated or connected thereto.

The optical sensor can be implemented as any electro-optical sensor which is arranged to convert light, or a change in light, into an electronic signal, or sensor signal. Light will be understood as electromagnetic radiation from the infrared, IR, visual, VIS, or ultraviolet, UV, spectral range. Furthermore, there may be more than a single optical sensor. For example, a first optical sensor may be complemented with a second optical sensor. The second optical sensor may include a proximity sensor, color sensor, or spectral sensor, and provide additional information to complement an endoscopic application, such as position, color or spectroscopic information, respectively. However, instead of a second optical sensor there may be an additional sensor of different type, e.g. a chemical sensor, temperature, gas, flow and/or pressure sensor etc.

The semiconductor laser comprises a laser diode, for example. Examples include Vertical-cavity surface-emitting lasers, VCSELs, or Vertical external-cavity surface-emitting lasers, VECSELs, to name just two possible implementations.

The transmission unit can be implemented in different ways. For example, the transmission unit and the optical sensor can be separate components. However, the optical sensor may comprise the transmission unit. The transmission unit is arranged to receive a sensor signal which is generated by the optical sensor. Furthermore, the transmission unit is arranged to drive the semiconductor laser to provide, at the optical interface, an optical signal which is representative of the sensor signal.

For example, the sensor arrangement is used in an endoscope which illuminates a part within a human body. The optical sensor collects light and generates the sensor signal. Typically, the sensor signal is an electronic signal. This signal is applied to the transmission unit where is used to drive the semiconductor laser. The transmission unit may be arranged to use the sensor signal directly to drive the semiconductor laser. However, the transmission unit may also be arranged to convert the sensor signal into a control signal which then is used to drive the semiconductor laser. For example, the control signal may be modulated such that it carries binary information which allows to reconstruct the sensor signal from the optical signal to be generated by the semiconductor laser. The optical signal is provided at the optical interface from where it can be guided to a receiver or a processing unit. The optical signal constitutes electromagnetic radiation from the IR, VIS, or UV spectral range depending on the emission characteristics of the semiconductor laser.

The proposed sensor arrangement enables the use of higher resolution and/or higher frame rates in endoscopic applications, e.g. using image sensors used in micro camera modules, while maintaining low cost. The use of the optical signal generated by a semiconductor laser allows for communicating the sensor signals over a large distance with reduced impact of signal degradation and noise, i.e. improves signal-to-noise ration. Integration of the semiconductor laser largely removes the bandwidth limitation of electrical cables currently used in the endoscopy field. Furthermore, electrical cable typically introduce noise in the sensor signal by the data transmission which is prone to strong electrical coupling between each wire in the cable. The proposed sensor arrangement opens up possibilities of using the sensor unit in other applications, such as smart clothes and augmented reality.

In at least one embodiment the sensor arrangement further comprises a communication cable. The communication cable comprises an optical waveguide which is coupled to the optical interface. For example, the communication cable comprises one or more optical fibers, such as a plastic or polymer optical fiber, POF. For example, the optical interface is arranged to be connected to the communication cable or optical waveguide.

The optical signal generated by the semiconductor laser is provided at the optical interface and then transmitted by way of the optical waveguide. In other words, the transmission unit drives the semiconductor laser such as to convert the sensor signal into the optical signal. The optical signal being representative of the sensor signal is transmitted by way of the optical waveguide to allow for data transmission to a receiver.

Using an optical waveguide such as a POF as physical medium for image data transmission solves the issue of limited bandwidth inherent to cheap, thin copper cables which are typically used in endoscopy. In addition, signal integrity will be much better with POF than with a copper cable. The communication cable suing an optical waveguide, such as POF, can be thin, lightweight and flexible. These are all benefits for medical endoscopy applications. While an optical waveguide in itself might not be cheaper, the fact that it enables higher data rates will be beneficial for the medical sensors. One considerable disadvantage overcome is the supply noise induced on the sensor's supply by the cable crosstalk.

In at least one embodiment the sensor unit is disposable. The coupling of the communication cable to the optical interface is arranged to be detachable. In addition, or alternatively, the coupling of the communication cable to the optical interface is arranged to be permanent. For example, the connection can be established by means of an optical connector of the communication cable, e.g. an optical fiber connector arranged at one end of the optical fiber. The optical interface may comprise a corresponding optical coupler. However, the communication cable or optical waveguide can be connected to the optical interface without a dedicated connector. For example, the communication cable or optical waveguide can be glued together to establish a more permanent connection. Depending on the intended use parts of the sensor arrangement can be configured for single or multiple use. For example, in order to meet medical hygiene standards an endoscope, or parts thereof, are only allowed to be used once. Arranging a detachable communication cable may help to reduce cost and increase flexibility.

In at least one embodiment the communication cable is split into the optical waveguide and at least one electrical cable. The optical waveguide is connected to the optical interface. Furthermore, the at least one electrical cable is electrically coupled to a power and/or data interface of the sensor unit. The communication cable may comprise one or more cores of optical waveguides. Furthermore, the communication cable may be complemented with the at least one electrical cable, such as copper cable, to also provide means for power supply and/or additional data communication, e.g. to transmit data which different from the sensor signal, such as configuration of the sensor. For example, in a direction from the receiving unit to the sensor, only a small amount of data may be transmitted for the configuration of the sensor. This can managed via a cheap electrical cable.

This way there may be a single cable which provides the necessary electrical connectivity when the sensor arrangement is used in an endoscope, for example, or any device where the sensor unit is supposed to be spaced apart from a receiver. In fact, the communication cable allows for separation of sensor unit and receiver over a distance determined by the length of the communication cable. Furthermore, additional electronic components which support operation of the sensor unit may be moved to a receiving unit in order to further reduce space requirements of the sensor unit. Such additional electronic components may comprise a signal processing unit, reference clock generator, or control unit to name but a few. In at least one embodiment a sensor unit comprises a carrier. At least the optical sensor, semiconductor laser and the transmission unit are electrically coupled to the carrier. In general, the carrier mechanically supports and electrically connects electronic or electrical components of the sensor unit, e.g. optical sensor, semiconductor laser and the transmission unit, using conductive tracks, terminals, bumps, redistribution layers, pads and/or through-substrate vias, for example. The carrier may be implemented as a printed circuit board, PCB, such as a flexible PCB. The carrier may be a microchip or a part thereof, e.g. with a redistribution layer, RDL, arranged as a metal layer on a surface of the microchip to make the components of the sensor unit (e.g. implemented as integrated circuits) electrically available, e.g. via the communication cable.

In at least one embodiment the carrier comprises a printed circuit board, which may be implemented as a flexible PCB, for example. The printed circuit board comprises a first section and a second section. The optical sensor is arranged on and electrically coupled to a first surface of the first section. The semiconductor laser is arranged on and electrically coupled to a second surface of the first section. The second surface is further coupled to the communication cable by way of a power interface.

The different sections and surfaces of the printed circuit board allow for a high degree of freedom when designing the sensor unit. For example, the first and the second sections may be tilted with respect to each other, such that the printed circuit board has an L-shape. A cross section of the first section basically defines a cross section of the sensor unit. For example, the first surface having the optical sensor can be arranged such that the optical sensor covers the larger part or all of the first section. Additional sensor electronics, e.g. the transmission unit, can be moved away from a light sensitive area of the optical sensor, e.g. the second surface of the first section. This way the cross section of sensor unit and, thus, the sensor arrangement, can be kept small. This is a benefit for endoscopic applications, for example. Small profile endoscopes help improving medical operations and lend medical personal more flexibility while operating. In addition the operation becomes less invasive for the patient.

In at least one embodiment the sensor arrangement further comprises a receiving unit. The receiving unit is operable to receive the optical signal via another optical interface. Furthermore, the receiving unit is operable to convert the optical signal into an electrical signal which is representative of the sensor signal generated by the optical sensor.

The sensor unit acts like a transmitter which collects the sensor signal by means of the optical sensor, converts the sensor signal into the optical signal by means of the transmission unit and the semiconductor laser. The optical signal is transmitted via the optical interface and through the communication cable. Finally, the optical signal is received by the receiving unit via the optical interface of the receiving unit.

For example, the receiving unit comprises a processing unit, such as a micro controller, to convert the optical signal into the representative electrical signal. Furthermore, the receiving unit may comprise a power supply to provide power via the communication cable to the sensor unit. For example, one or more of additional electrical cables, such as copper cables, can be used for power supply. The receiving unit can be implemented as a reusable base station. This way more expensive electronics, such as the processing unit can be used multiple times in order to reduce overall cost of operation of the sensor arrangement.

In at least one embodiment the sensor unit and the receiving unit are interconnected by means of the communication cable. The connection is arranged such that the optical waveguide is coupled to an input of the receiving unit. The communication cable allows for optical communication of electromagnetic radiation (optical signal) between the sensor unit and the receiving unit. Optical type of transmission is much less likely to interfere with electrical data and/or power supply, thus, improving signal-to-noise ratio.

In at least one embodiment the sensor comprises at least one imaging sensor. In addition, or alternatively, the sensor unit comprises a micro camera having the at least one image sensor. The imaging sensor and/or micro camera may generate a large amount of data. Typically, a common electrical cable, such as a copper cable, may not provide sufficient bandwidth to transmit the data from the sensor unit, e.g. to the receiving unit. However, the communication cable due to its optical type transmission allows for an increased higher bandwidth

In addition, or alternatively, the transmission unit and the at least one imaging sensor are arranged on a same chip.

In at least one embodiment the transmission unit is arranged to drive the semiconductor laser to transmit a sequence of optical signals. The sequence of optical signals is representative of the sensor signals generated by rows and/or columns of the at least one imaging sensor. The receiving unit is operable to reconstruct an image from the sequence of optical signals generated by the imaging sensor. In other words, the sensor arrangement is configured to capture images, e.g. from within a human body when used in an endoscope, and transmit the image as sequences of optical signals. The sequence of optical signals can be collected, e.g. by the receiving unit, and allow to reconstruct said image. Transmission of the image is done optically and, thus, is less prone to electrical interference.

In at least one embodiment an endoscope comprises a distal end which is interconnected with a proximal end. The endoscope comprises at least one sensor arrangement according to the concept laid out above. The distal end comprises the sensor unit. The improved concept in all aspects discussed so far can be applied to or implemented as an endoscope. For example, the sensor unit may be integrated into a module which is arranged to be inserted into a human body for diagnosis.

In at least one embodiment the proximal end comprises the receiving unit. The distal end and the proximal end are interconnected by means of the communication cable. The optical waveguide is coupled to the input of the receiving unit. For example, the module can be operated and data transmission can be received by the receiving unit, or base station, by means of the communication cable.

In at least one embodiment the optical waveguide is coupled to the optical interface and is operable to transmit the optical signal in a unidirectional manner. The at least one electrical cable is arranged for power supply and/or bidirectional communication between the distal and the proximal end.

The communication cable may be split into the optical waveguide, such as an optical fiber, and the at least one electrical cable. This separation further reduces crosstalk between unidirectional transmission of the optical signal, i.e. the sensor signal, and other transmission of data (bidirectional) and/or power supply. One aspect of this separation relies on optical communication of electromagnetic radiation (optical signal) and electrical communication of electrical signals (bidirectional data transmission and/or power supply). These different types of transmissions are much less likely to interfere, thus, improving signal-to-noise ratio.

In at least one embodiment a method of operating a sensor arrangement comprising a sensor unit further having an optical sensor, a semiconductor laser connected to an optical interface and a transmission unit, comprises the following steps. First, a sensor signal is generated using the optical sensor. The sensor signal is received by means of the transmission unit. The semiconductor laser is driven by means of a transmission unit and provides, at the optical interface, an optical signal which is representative of the sensor signal.

For example, the sensor arrangement is used in an endoscope which illuminates a part within a human body. The optical sensor collects light and generates the sensor signal. Typically, the sensor signal is an electronic signal. This signal is applied to the transmission unit where is used to drive the semiconductor laser. The transmission unit may be arranged to use the sensor signal directly to drive the semiconductor laser. However, the transmission unit may also be arranged to convert the sensor signal into a control signal which then is used to drive the semiconductor laser. For example, the control signal may be modulated such that it carries binary information which allows to reconstruct the sensor signal from the optical signal to be generated by the semiconductor laser. The optical signal is provided at the optical interface from where it can be guided to a receiver or a processing unit. The optical signal constitutes electromagnetic radiation from the IR, VIS, or UV spectral range depending on the emission characteristics of the semiconductor laser.

The proposed method of operating a sensor arrangement allows for higher resolution and/or higher frame rates in endoscopic applications, e.g. using image sensors used in micro camera modules, while maintaining low cost. The use of the optical signal generated by a semiconductor laser allows for communicating the sensor signals over a large distance with reduced impact of signal degradation and noise, i.e. improves signal-to-noise ration. Integration of the semiconductor laser largely removes the bandwidth limitation of electrical cables currently used in the endoscopy field. Furthermore, electrical cable typically introduce noise in the sensor signal by the data transmission which is prone to strong electrical coupling between each wire in the cable. The proposed sensor arrangement is likely to open up possibilities of using the sensor unit in other applications, such as smart clothes and augmented reality.

In at least one embodiment the sensor arrangement further comprises a communication cable interconnecting a distal end of an endoscope, having the sensor unit, to a proximal end of the endoscope. The method comprises the further step of transmitting the optical from the distal end to the proximal end.

The discussion of the improved concept focused on optical sensors and/or optical image sensors so far. However, the improved concept is not restricted to this type of sensors but can be extended to other types of sensors. If not stated otherwise all features discussed above with respect to the sensor arrangement having an optical sensor can be applied mutatis mutandis to a sensor arrangement having other types of sensors. Examples of other types of sensors include imaging applications requiring to bridge a certain distance (1 to 2m, for example) between image sensor and a receiving unit (a data processor, for example), e.g. for augmented reality and smart clothing applications. Another example relates to magnetic resonance imaging, MRI. MRI employs coils which are configured to receive radio waves and have to be placed in strong magnetic fields of some 1.5 Tesla and are expected to be placed in 3 Tesla or even more in the near future. To not disturb the magnetic field and decrease the image quality it's may prove important to keep metallic materials (eddy currents) away as much as possible. Other medical applications also involve imaging in other ranges or types of radiation.

In at least one embodiment a sensor arrangement with a sensor unit comprises a sensor and a semiconductor laser which is connected to an optical interface. Furthermore, the sensor arrangement comprises a transmission unit. The transmission unit is arranged to receive a sensor signal generated by the sensor. Furthermore, the transmission unit is arranged to drive the semiconductor laser to provide at the optical interface an optical signal which is representative of the sensor signal.

The sensor can be implemented as any electro-optical sensor which is arranged to convert radiation, or a change in radiation, into an electronic signal, or sensor signal. Radiation may include light will be understood as electromagnetic radiation from the infrared, IR, visual, VIS, or ultraviolet, UV, spectral range. Others types of radiation may include X-rays or radio waves, for example. Furthermore, there may be more than a single sensor or additional sensor of different type implemented in the sensor unit.

The semiconductor laser comprises a laser diode, for example. Examples include Vertical-cavity surface-emitting lasers, VCSELs, or Vertical external-cavity surface-emitting lasers, VECSELs, to name just two possible implementations.

The transmission unit can be implemented in different ways. For example, the transmission unit and the sensor can be separate components. However, the sensor may comprise the transmission unit. The transmission unit is arranged to receive a sensor signal which is generated by the sensor. Furthermore, the transmission unit is arranged to drive the semiconductor laser to provide, at the optical interface, an optical signal which is representative of the sensor signal. The sensor collects radiation and generates the sensor signal. Typically, the sensor signal is an electronic signal. This signal is applied to the transmission unit where is used to drive the semiconductor laser. The transmission unit may be arranged to use the sensor signal directly to drive the semiconductor laser. However, the transmission unit may also be arranged to convert the sensor signal into a control signal which then is used to drive the semiconductor laser. For example, the control signal may be modulated such that it carries binary information which allows to reconstruct the sensor signal from the optical signal to be generated by the semiconductor laser. The optical signal is provided at the optical interface from where it can be guided to a receiver or a processing unit. The optical signal constitutes electromagnetic radiation from the IR, VIS, or UV spectral range depending on the emission characteristics of the semiconductor laser.

The proposed sensor arrangement enables the use of higher resolution and/or higher frame rates in sensor or imaging applications, e.g. using image sensors used in micro camera modules, while maintaining low cost. The use of the optical signal generated by a semiconductor laser allows for communicating the sensor signals over a large distance with reduced impact of signal degradation and noise, i.e. improves signal-to-noise ration. Integration of the semiconductor laser largely removes the bandwidth limitation of electrical cables currently used in parallel with the sensor unit. Furthermore, electrical cables typically introduce noise in the sensor signal by the data transmission which is prone to strong electrical coupling between each wire in the cable. The proposed sensor arrangement is likely to open up possibilities of using the sensor unit in various applications, where sensor unit and receiver are spaced apart, such as smart clothes and augmented reality.

For example, using an optical wave guide (e.g. POF, fiber) allows for transmitting large amount of data from the sensor to a receiving unit such as an external control unit. Electrical wires can be used to supply the sensor. The supply voltage on those wires can modulated to transmit a relative small amount of data from the control unit to the sensor (e.g. for sensor configuration). A small amount of data can transmitted from the sensor to the control unit in parallel to the high-speed optical data transmission by modulating the supply current (e.g. for separate status information or testing). In parallel to the supply wires separate wires can used for slower communication from and/or to the sensor in parallel to the optical transmission. As an option the transmission unit can be directly integrated into the sensor.

In at least one embodiment the sensor arrangement further comprises a communication cable. The communication cable comprises an optical waveguide which is coupled to the optical interface. The optical signal is transmitted by way of the optical waveguide. For example, the communication cable comprises one or more optical fibers, such as a plastic or polymer optical fiber, POF. For example, the optical interface is arranged to be connected to the communication cable or optical waveguide.

The optical signal generated by the semiconductor laser is provided at the optical interface and then transmitted by way of the optical waveguide. In other words, the transmission unit drives the semiconductor laser such as to convert the sensor signal into the optical signal. The optical signal being representative of the sensor signal is transmitted by way of the optical waveguide to allow for data transmission to a receiver.

Using an optical waveguide, such as a POF, as physical medium for image data transmission solves the issue of limited bandwidth inherent to cheap, thin copper cables which may be used as an alternative. In addition, signal integrity will be much better with POF than with a copper cable. The communication cable using an optical waveguide, such as POF, can be thin, lightweight and flexible. While an optical waveguide in itself might not be cheaper, the fact that it enables higher data rates will be beneficial for many imaging applications. One considerable disadvantage overcome is the supply noise induced on the sensor's supply by the cable crosstalk.

In at least one embodiment the sensor arrangement further comprises a receiving unit which is operable to receive the optical signal via the optical interface.

In at least one embodiment the sensor unit and the receiving unit are interconnected by means of the communication cable. The interconnection is arranged such that the optical waveguide is coupled to an input of the receiving unit.

In at least one embodiment the receiving unit is operable to convert the optical signal into an electrical signal which is representative of the sensor signal generated by the sensor.

In at least one embodiment the sensor comprises at least one imaging sensor.

In at least one embodiment the transmission unit is arranged to drive the semiconductor laser to transmit a sequence of optical signals. The sequence of optical signals is representative of the sensor signal generated by the at least one imaging sensor.

In at least one embodiment the receiving unit is operable to reconstruct an image from the sequence of optical signals generated by the imaging sensor.

In at least one embodiment a first module comprises the sensor unit and a carrier. The sensor, semiconductor laser and the transmission unit are arranged contiguous with and electrically coupled to the carrier. In addition, or alternatively, a second module comprises the receiving unit.

In at least one embodiment the communication cable interconnects the first and the second module. The optical waveguide is coupled to the optical interface and operable to transmit the optical signal in a unidirectional manner. Furthermore, the communication cable comprises one or more vias which are arranged for power supply and/or bidirectional communication between the first and the second module.

In at least one embodiment an imaging device comprises at least one sensor arrangement according to the concept discussed above.

In at least one embodiment the imaging device is arranged as a camera for augmented reality applications. In another embodiment the imaging device is arranged as part of a smart clothing.

In at least one embodiment a medical device comprises at least one sensor arrangement according to the concept discussed above.

In at least one embodiment the medical device comprises an endoscope, an x-ray radiograph, a magnetic resonance imager, a medical ultra sonograph and/or a computer tomograph.

In at least one embodiment a method of operating a sensor arrangement comprising a sensor unit further having a sensor, a semiconductor laser connected to an optical interface and a transmission unit, comprises the following steps. First, a sensor signal is generated using the sensor. A sensor signal is received by means of the transmission unit. Finally, the transmission unit drives the semiconductor to provide, at the optical interface, an optical signal which is representative of the sensor signal.

Further implementations of the method of operating a sensor arrangement are readily derived from the various implementations and embodiments of the sensor arrangement, sensor arrangement for an endoscope, endoscope, imaging device and medical device discussed above, and vice versa.

In the following, the concept presented above is described in further detail with respect to drawings, in which examples of embodiments are presented. In the embodiments and Figures presented hereinafter, similar or identical elements may each be provided with the same reference numerals. The elements illustrated in the drawings and their size relationships among one another, however, should not be regarded as true to scale, rather individual elements, such as layers, components, and regions, may be exaggerated to enable better illustration or a better understanding.
- Figure 1: shows an example embodiment of a sensor arrangement,
- Figure 2: shows an example embodiment of a sensor with ball grid array,
- Figure 3: shows an example embodiment of an interface, and
- Figure 4: shows another example embodiment of a sensor arrangement.

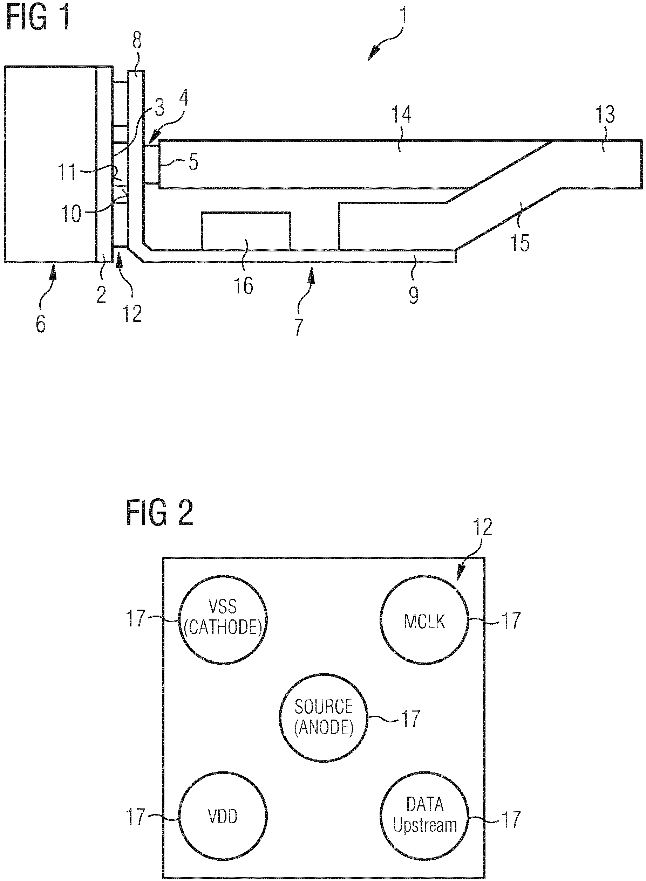

Figure 1 shows an example embodiment of a sensor arrangement. The sensor arrangement comprises a sensor unit 1. The sensor unit 1 further comprises a sensor 2, a transmission unit 3, and a semiconductor laser 4 which is connected to an optical interface.

The sensor unit 1 can be configured with different types of sensors. Examples include optical sensors, especially image sensors. However, the sensor unit 1 can also be equipped with non-optical sensors, such as magnetic resonance imaging coils. In the following the sensor is an image sensor and the sensor arrangement is arranged for an endoscope. Further examples, i.e. an MRI and computer tomograph will be discussed later on. In general, the sensor arrangement can be applied to different types of sensors and applications. One field which may benefit from the proposed concept is given by sensors which are spaced apart from a receiving unit and interconnected to the unit, or base, by some sort of communications cable. Such examples include optical sensors and image sensors for augmented reality and smart clothing.

In this embodiment the sensor arrangement is arranged for an endoscope. The sensor unit 1 is embedded in a module which may be disposable. The module is intended for medical use and seals the sensor unit when inserted into a human body. The sensor 2 is an image sensor, e.g. a CMOS image sensor or CCD. In this embodiment the module comprises optics 6, such as a lens or lens system. This way the module constitutes a micro-camera module. A backside of the image sensor is connected to a ball grid array.

The sensor unit 1 further comprises a printed circuit board 7. The printed circuit board is implemented as a flexible PCB, with a first section 8 and a second section 9. The two sections are tilted such that the printed circuit board 7 has an L-shape. The image sensor is arranged on a first surface 10 of the first section 8. Furthermore, the image sensor is electrically connected to the printed circuit board 7 by means of the ball grid array 12.

The semiconductor laser 4 is arranged contiguous with a second surface 11 of the printed circuit board 7. Furthermore, the semiconductor laser 4 is electrically connected to the image sensor by means of the ball grid array 12. In this embodiment the semiconductor laser 4 comprises a laser diode, such as a Vertical-cavity surface-emitting lasers, VCSEL, or Vertical external-cavity surface-emitting lasers, VECSEL.

The transmission unit 3 can be implemented in different ways. In this example, the image sensor and the transmission unit are integrated on a same chip. The transmission unit has at least two functions. First, it receives a sensor signal or sequence of sensor signals, such as image data, generated by the image sensor. Second, the transmission unit is arranged to drive the semiconductor laser to provide, at the optical interface, an optical signal which is representative of the sensor signal.

The semiconductor laser 4 is connected to the optical interface 5 which is arranged on the second surface 11 of the first section 8, opposite the first surface 10. For example, the optical interface comprises an optical coupler. The optical coupler is coupled to an optical waveguide of a communication cable 13. The communication cable comprises the optical waveguide 14, which in this embodiment is implemented as one or more optical fibers, such as polymeric optical fiber, or plastic optical fiber, POF. Furthermore, the communication cable 14 comprises one or more electrical cables 15, such as copper cable.

Coupling of the communication cable 13 to the optical interface 5 can be permanent or detachable. For example, an optical connector of the communication cable, such as an optical fiber connector is arranged at one end of the optical fiber, and fits to the optical coupler of the optical interface. However, the communication cable or optical waveguide can be connected to the optical interface without a dedicated connector. For example, the communication cable or optical waveguide can be glued together, e.g. by means of a polymer, to establish a more permanent connection. Depending on the intended use parts of the sensor arrangement can be configured for single or multiple use. For example, in order to meet medical hygiene standards for an endoscope, or parts thereof, are only allowed to be used once. Arranging a detachable communication cable may help to reduce cost and increase flexibility.

The communication cable 13 in this embodiment also comprises the electrical cables 15. These electrical cables serve a different purpose than the optical waveguide. For example, three electrical cables are provided to supply power to the sensor unit, e.g. VSS (cathode), source (anode) and VDD. Furthermore, another cable MCLK is provided to receive an external clock signal, e.g. from a receiving unit. Yet another electrical cable is provided for data transmission. The electrical cables 15 are connected to the printed circuit board 7. For example, the printed circuit board comprises an interface, e.g. a power interface, to which the electrical cables can be connected. The connection can be permanent or detachable similar to the optical waveguide. The cables and interface have corresponding connectors or may be permanently connected to each other. A capacitor 16 is arranged on and electrically connected to a second surface of the printed circuit board. The capacitor is arranged to decouple the power supply.

In operation the image sensor collects light and generates the sensor signal (or a sequence of sensor signals). Typically, the sensor signal is an electronic signal and resembles an image taken by the image sensor. This signal is applied to the transmission unit where is used to drive the semiconductor laser. The transmission unit may be arranged to use the sensor signal directly to drive the semiconductor laser. However, the transmission unit may also be arranged to convert the sensor signal into a control signal which then is used to drive the semiconductor laser. For example, the control signal may be modulated such that it carries binary information which allows to reconstruct the sensor signal.

The optical signal is provided at the optical interface 5 from where it can be guided to an external receiving or processing unit (not shown). The optical signal constitutes electromagnetic radiation from the IR, VIS, or UV spectral range depending on the emission characteristics of the semiconductor laser. The communication cable is split into the optical waveguide and electrical cables. This allows for different means of data transmission. Transmission of the optical signal via the optical interface is unidirectional. This way the optical interface, semiconductor laser and transmission unit can be optimized to transmit with high bandwidth and high data rates. Other transmission of data may be bidirectional and, at lower bandwidth and data rates may involve configuration data, clock etc. Transmission of such data does not need high bandwidth and high data rates and is prone to interferences and crosstalk.

Figure 2 shows an example embodiment of a sensor with ball grid array. The drawing depicts the ball grid array 12 which is connected to the image sensor. The ball grid array comprises several pins 17 to electrically connect the image sensor to the communication cable. For example, there are dedicated pins for power supply VSS (cathode) and VDD and another connected to source (anode) of the image sensor. Furthermore, another pin is arranged to receive a clock from an electrical cable of the communication cable. Using the ball grid array allows for electrically contacting the image sensor from its backside and thereby allow for a compact cross-section of the image sensor. The electrical pins 17 can be arranged to supply the sensor unit with power. For this purpose two electrical cables may suffice. It will be also configured and controlled with this two lines by modulating the supply voltage (receiving unit to sensor unit) and modulating the supply current (sensor unit to receiving unit).

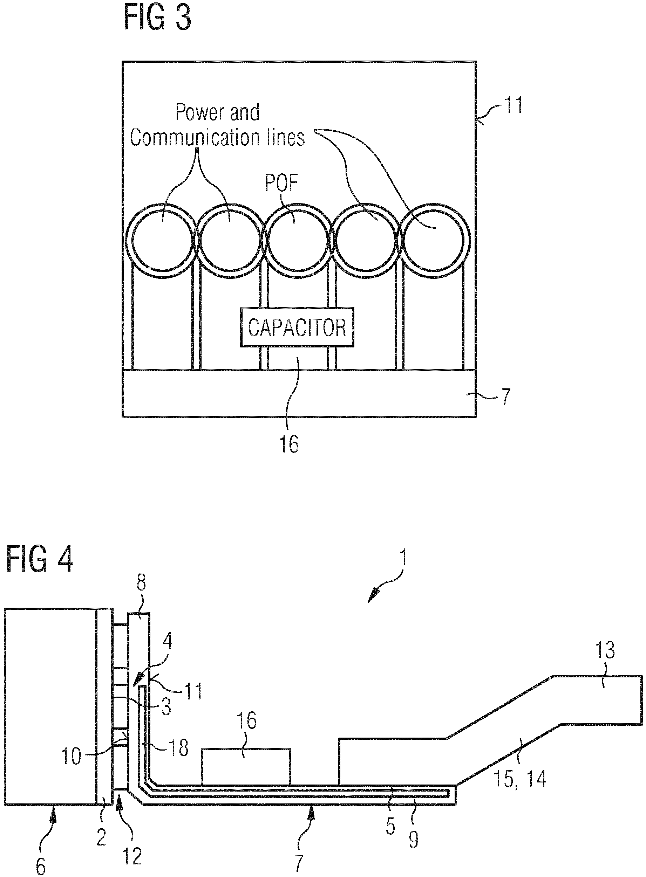

Figure 3 shows an example embodiment of an interface. The drawing shows a view from communication cable to the printed circuit board. The interface is depicted with four connectors, e.g. power supply VSS (cathode) and VDD. Furthermore, another connector is arranged to connect to the cable carrying the clock signal. Another connector is arranged to connect to the electrical cable arranged for bidirectional data transmission. The connector in the middle is arranged to plug into or couple the optical waveguide for unidirectional transmission of the sensor signal.

One possible implementation of the sensor arrangement could be summarized as follows.
- an image sensor connected to a ball grid array with 5 pins, one pin would control the semiconductor laser,
- communication cable with four electrical wires and an optical waveguide line,
- semiconductor laser coupled in the printed circuit board, e.g. in the back of the image sensor on top of the control pin to control the semiconductor laser, e.g. on the middle of the package,
- flexible PCB with semiconductor laser embedded to allow connection of the four remaining pins to the electrical wires, and if necessary assemble a decouple capacitor between power wires (VDD and VSS),
- a tip of the flex PCB (first section) that holds the semiconductor laser may have a hole to assembly the waveguide directly to the semiconductor laser, or by use of a polymer guide hole fabricated with standard UV lithography so that the optical fiber can be directly inserted on the hole.
- As alternative, the PCB could have an optical waver guideline to redirect the optical signal from the semiconductor laser to the optical waveguide.

Figure 4 shows another example embodiment of a sensor arrangement. This embodiment is based on the one shown in Figure 1. However, an optical waver guideline is arranged contiguous with the first and second sections of the printed circuit board. The optical waver guideline connects the semiconductor laser with the interface. The optical waver guideline is arranged to redirect the optical signal from the semiconductor laser, e.g. VCSEL, to the optical waveguide, e.g. optical fiber.

The sensor arrangement can be used for an endoscope. In this application the sensor unit is comprised by the module discussed above. The communication cable is connected to a receiving unit or base station (not shown). The full module and communication cable can be disposable. The communication cable is connected to a base station which is not disposable. However, the coupling of the communication cable to the sensor unit might be permanent. In that case the sensor unit will also need to be disposable. Any electronics after the sensor unit will not be disposable as they are part of the medical equipment that is re-used. For example, the module can be delivered with an optical connector on the optical waveguide, e.g. optical fiber, and electrical connectors for the electrical (supply and low speed data connection up to sensor) cable.

The proposed sensor arrangement for endoscopy allows for higher resolution, better quality and low cost modules for the medical field. It can also be used in the consumer market where micro camera modules need to be placed in small spaces with no room for placing the adjacent electronics. Adjacent electronics can be placed at longer distances, and the communication to the module or sensor unit is made by the use of the optical waveguide, which requires very little space.

Besides medical endoscopy, esp. disposable endoscopy, other applications may benefit from the proposed sensor arrangement, especially where there is a long data connection required between sensor and base station. Currently, the communication medium is copper wire, which has bad signal transmission characteristics because of the application requiring cheap and thin cables. Even with a better cable the bandwidth of a copper cable is limited to short distances. Other applications may require to bridge a certain distance (1 to 2m) between sensor and data processor, e.g. augmented reality and smart clothing.

In another example embodiment all or some components of the sensor unit are arranged and electrically connected to a redistribution layer at the back of the sensor. There may be no printed circuit board.

In another example embodiment the sensor is arranged for magnetic resonance imaging, MRI. MRI employs coils which are configured to receive radio waves and have to be placed in strong magnetic fields of some 1.5 Tesla and are expected to be placed in 3 Tesla or even more in the near future. To not disturb the magnetic field and decrease the image quality it's may prove important to keep metallic materials (eddy currents) away as much as possible. Other medical applications also involve imaging in other ranges or types of radiation. Besides the different type of sensor the remaining components could be implemented as depicted above with respect to Figures 1 to 3.

In another example embodiment the sensor is arranged for computed tomography, CT. The sensor may comprise a scintillator which is arranged to convert X-ray radiation into light. Light may then be detected by the image sensor and sensor arrangement discussed in Figures 1 to 3.

In another example embodiment the sensor is arranged for smart clothing and/or augmented reality. For example, the sensor arrangement discussed in Figures 1 to 3 is connected to a clothing. The image sensor is attached to the clothing at a first position and, via the communication cable, connected to a receiving unit which is located at a remote second position. Thus, detection and processing are separated. In fact, the sensor unit can be implemented in a compact way while the receiving unit may not need to be compact. The receiving unit can be worn in a pocket or at a position of the clothing which provides convenient space. Augmented reality may be a feature of the receiving unit, i.e. is subject to processing. The receiving unit may be coupled to glasses worn be a user, e.g. by means of a wireless network, in order to provide the sensor signal and a processed augmented reality image to the user.

One possible implementation of the sensor arrangement could be summarized as follows.
- Using a single optical wave guide (e.g. POF, fibre) to transmit large amount of data from the sensor to an external control unit
- Using electrical wires to supply the sensor
- The supply voltage on those wires can be modulated to transmit a relative small amount of data from the control unit to the sensor (e.g. for sensor configuration)
- A small amount of data can be transmitted from the sensor to the receiving unit in parallel to the high-speed optical data transmission by modulating the supply current (e.g. for separate status information or testing)
- In parallel to the supply wires separate wires can be used for slower communication from and/or to the sensor in parallel to the optical transmission
- As an option the driver (or transmission unit) for the light source (e.g. VCSEL) can be directly integrated into the sensor chip.

All implementations discussed above share some common benefits. Sensors or a set of sensors like e.g. micro-cameras or MRI coils are generating a big amount of data. To transmit them from the sensor to the receiving unit a robust, small, low cost interface will be beneficial. The proposed sensor arrangement combines robust high speed data transmission, higher resolution and/or higher frame rates in image sensors, e.g. used in micro camera modules, while maintaining low cost. This opens up possibilities of using the sensor arrangement in various imaging applications such as smart clothes, augmented reality. Electromagnetic disturbance can be reduced and electromagnetic/magnetic sensitivity can be increased. Furthermore, the concept can be implemented with a simple interface with minimized number of connections. High-speed data transmission may involve a single direction with a minimized interface for one optical waveguide and two electrical wires, for example.

### Reference numerals

- 1: sensor unit
- 2: sensor, optical sensor
- 3: transmission unit
- 4: semiconductor laser
- 5: optical interface
- 6: optics
- 7: printed circuit board, carrier
- 8: first section
- 9: second section
- 10: first surface
- 11: second surface
- 12: ball grid array
- 13: communication cable
- 14: optical waveguide
- 15: electrical cable
- 16: capacitor
- 17: pins of BGA
- 18: optical waver guideline

## Claims

1. A sensor arrangement with a sensor unit (1) comprising:
- a sensor (2),
- a semiconductor laser (4) connected to an optical interface (5),
- a transmission unit (3);
wherein:
- the transmission unit (3) is arranged to receive a sensor signal generated by the sensor (2) and drive the semiconductor laser (4) to provide at the optical interface (5) an optical signal representative of the sensor signal.

2. The sensor arrangement according to claim 1, further comprising a communication cable (13), wherein:
- the communication cable (13) comprises an optical waveguide (14) coupled to the optical interface (5), and
- the optical signal is transmitted by way of the optical waveguide (14).

3. The sensor arrangement according to claim 1 or 2, further comprising a receiving unit operable to receive the optical signal via the optical interface (5).

4. The sensor arrangement according to claim 3, wherein the sensor unit (1) and the receiving unit are interconnected by means of the communication cable (13), such that the optical waveguide (14) is coupled to an input of the receiving unit.

5. The sensor arrangement according to claim 4, wherein the receiving unit is operable to convert the optical signal into an electrical signal representative of the sensor signal generated by the sensor.

6. The sensor arrangement according to one of claims 1 to 4, wherein the sensor (2) comprises at least one imaging sensor.

7. The sensor arrangement according to claim 6, wherein
- the transmission unit (3) is arranged to drive the semiconductor laser (4) to transmit a sequence of optical signals, and
- the sequence of optical signals is representative of the sensor signal generated by at least one imaging sensor.

8. The sensor arrangement according to claim 5 or 6, wherein the receiving unit is operable to reconstruct an image from the sequence of optical signals generated by the imaging sensor.

9. The sensor arrangement according to one of claims 3 to 8, wherein:
- a first module comprises the sensor unit (1) and a carrier (7), wherein the sensor (1), semiconductor laser (4) and the transmission unit (3) are arranged contiguous with and electrically coupled to the carrier (7), and/or
- a second module comprises the receiving unit.

10. The sensor arrangement according to claim 9, wherein
- the communication cable (13) interconnects the first and the second module,
- the optical waveguide (14) coupled to the optical interface (5) is operable to transmit the optical signal in a uni-directional manner, and
- the communication cable (13) comprises one or more wires (15) arranged for power supply and/or bi-directional communication between the first and the second module.

11. An imaging device, comprising at least one sensor arrangement according to one of claims 1 to 10.

12. The imaging device according to claim 11, wherein the imaging device is arranged according to one of:
- a camera for augmented reality applications,
- a part of a smart clothing.

13. A medical device, comprising at least one sensor arrangement according to one of claims 1 to 10.

14. The medical device according to claim 13, the medical device comprises:
- an endoscope,
- an X-ray radiograph,
- a magnetic resonance imager,
- a medical ultra-sonograph, and/or
- a computer tomograph.

15. Method of operating a sensor arrangement comprising a sensor unit (1) further having a sensor (2), a semiconductor laser (4) connected to an optical interface (5) and a transmission unit (3), the method comprising the steps of:
- generating a sensor signal using the sensor (1),
- receiving the sensor signal by means of the transmission unit (3), and
- drive the semiconductor laser (4) by means of the transmission unit (3) to provide at the optical interface (5) an optical signal representative of the sensor signal.
